# EUROPEAN PATENT APPLICATION

(11) **EP 2 236 515 A1**
(43) Date of publication of application: **06.10.2010**
(21) Application number: 08864708.6
(22) Date of filing: 24.12.2008
(51) Int. Cl.: C07K 14/21, A61K 38/00, A61K 39/104, A61K 39/395, A61P 31/04, C07K 16/12, C12N 5/10, C12N 15/09, G01N 33/53, G01N 33/569, G01N 33/577, C12P 21/08

(54) **COMPONENT PROTEIN PA1698 FOR TYPE-III SECRETION SYSTEM OF PSEUDOMONAS AERUGINOSA**

(30) Priority: 25.12.2007 JP 2007331653
(71) Applicant: Meiji Seika Kaisha Ltd., Tokyo 104-8002 (JP)
(72) Inventor: KUMAGAI, Masashi, Yokohama-shi Kanagawa 222-8567 (JP); TANAKA, Jiro, Yokohama-shi Kanagawa 222-8567 (JP); NAGASO, Hiroshi, Yokohama-shi Kanagawa 222-8567 (JP); NINOMIYA, Tomohisa, Yokohama-shi Kanagawa 222-8567 (JP); OTSUKA, Keiko, Yokohama-shi Kanagawa 222-8567 (JP); AKABANE, Hirotomo, Yokohama-shi Kanagawa 222-8567 (JP); SUZUKI, Takahisa, Yokohama-shi Kanagawa 222-8567 (JP)
(74) Representative: Cohausz & Florack
(86) International application number: PCT/JP2008/073492
(87) International publication number: WO 2009/081955

(57) **Abstract**

An object is to provide an antibody and a vaccine composition which have an ability to practically prevent or treat a Pseudomonas aeruginosa infection, and which can cope with the diversity of clinical isolates derived from patients infected with Pseudomonas aeruginosa. According to the present invention, an antibody against a PA1698 protein that is a type III secretion system component protein of Pseudomonas aeruginosa or against a peptide of the protein, and a vaccine composition comprising the protein or the peptide are provided.

## Description

### Technical Field

The present invention relates to an antibody against a type III secretion system component protein PA1698 of Pseudomonas aeruginosa, as well as a pharmaceutical composition, a diagnostic agent for a Pseudomonas aeruginosa infection, a therapeutic agent for a Pseudomonas aeruginosa infection, and a kit for detecting Pseudomonas aeruginosa, which comprise the antibody. Moreover, the present invention relates to a vaccine composition comprising the PA1698 protein or a peptide thereof as an antigen.

### Background of the Invention

Pseudomonas aeruginosa (Pseudomonas aeruginosa) is a gram-negative bacillus widely and generally distributed in natural environments such as soil and water, and causes refractory and serious fatal infections. A main target thereof is infection susceptible patients (host) with attenuated biological defense mechanisms, including burned, organ-transplanted or cancer patients. Such patients are generally called compromised hosts. Pseudomonas aeruginosa is a major causative bacterium of hospital infections. Furthermore, the lung infections caused by this bacterium are fatal to cystic fibrosis patients. An antibacterial agent having an anti-Pseudomonas aeruginosa activity is mainly administered to these patients. However, sufficient therapeutic effects are not obtained in many cases due to the drug resistance of Pseudomonas aeruginosa. Alternatively, vaccines or antibodies directed against Pseudomonas aeruginosa have also been studied for years. However, the method directly using inactivated forms of the bacteria has disadvantages that various types of vaccines and antibodies have to be prepared individually for the respective serotypes of Pseudomonas aeruginosa.

Under such a situation, the prevention or treatment of a Pseudomonas aeruginosa infection has been expected through active or passive immunity acquired by using a protein derived from Pseudomonas aeruginosa, the protein having a common amino acid sequence among Pseudomonas aeruginosa strains. Known examples of a Pseudomonas aeruginosa-derived protein applied in the form of vaccines include: a recombinant protein in which portions of outer membrane proteins OprF and OprI are fused with each other (Japanese Unexamined Patent Application Publication No. Hei 8-245699) ; a type IV pilin protein (WO 2004/099250) ; and the like.

Therapeutic antibodies targeting a protein derived from Pseudomonas aeruginosa reported by now include: an anti-type IV pilin antibody (WO2004/099250); an anti-PA1706 (or PcrV) antibody (US 6309651, US 6827935); an anti-PA5158 antibody (WO 2007/049770); an anti-PA0427 antibody (WO2007/114340); and the like.

On the other hand, a bacteria-derived protein commonly possessed by clinical isolates of Pseudomonas aeruginosa which exhibit diverse serotypes is applicable as a "Pseudomonas aeruginosa common antigen" to the prevention, diagnosis or treatment of a Pseudomonas aeruginosa infection. Thus, such a protein has always been demanded.

Meanwhile, a PA1698 protein encoded by a PA1698 (or PopN) gene (Genebank accession No. AF010150) is a protein constituting a type III secretion system of Pseudomonas aeruginosa (Journal of Bacteriology, 2007, 189, 2599-2609). The type III secretion system is a system used when a pathogenic bacterium directly transports a pathogenic factor into a host cytoplasm. The type III secretion system is formed of approximately 30 different types of proteins. So far, the anti-PA1706 (or PcrV) antibody is reported to have a protective effect against infections (US6309651, US6827935).

However, to what degree the PA1698 protein mutates among strains is not known. There is no case where this protein or a peptide fragment thereof is used as a component of a vaccine, and where an antibody produced from this protein or a peptide fragment thereof is used as a therapeutic agent or diagnostic agent for infections.

### Disclosure of the Invention

An object of the present invention is to provide an antibody and a vaccine composition which have an ability to practically prevent or treat a Pseudomonas aeruginosa infection, and which can cope with the diversity of clinical isolates derived from patients infected with Pseudomonas aeruginosa.

In order to achieve the above object, the present inventors have attempted to search Pseudomonas aeruginosa-outer membrane proteins for a novel and useful "Pseudomonas aeruginosa common antigen". As a result of various studies, the present inventors have found by GeneChip analysis that a gene encoding a PA1698 (also known as PopN) protein, i.e., a type III secretion system component protein of Pseudomonas aeruginosa, is constantly expressed regardless of the presence or absence of human sera (Example 1). Moreover, by making gene analysis on 93 clinical isolates of Pseudomonas aeruginosa, the present inventors have surprisingly found that approximately 70% of the clinical isolates have the amino acid sequence of the PA1698 protein completely conserved, and that even remaining approximately 30% of the clinical isolates have only 1 or 2 amino acid mutations (Example 2). Furthermore, the present inventors have confirmed that an antibody obtained by immunization with a PA1698 recombinant protein binds to the PA1698 protein (Examples 7 to 9), that the antibody shows a cytotoxity suppression effect (Example 10), and that the antibody shows a potent protective effect against infections on Pseudomonas aeruginosa-infected model mice (Examples 11 and 12).

In other words, by the present invention, it has been found that the PA1698 protein, i.e. , the type III secretion system component protein of Pseudomonas aeruginosa, is useful as the Pseudomonas aeruginosa common antigen, and that the antibody against this antigen binds to Pseudomonas aeruginosa, thus exhibiting an excellent preventive effect against infections of Pseudomonas aeruginosa. In this manner, the present invention has been completed.

More specifically, the present invention relates to the following inventions.
<1> An antibody or a functional fragment thereof, which binds to a PA1698 protein derived from Pseudomonas aeruginosa.
<2> The antibody or the functional fragment thereof according to <1>, wherein the PA1698 protein derived from Pseudomonas aeruginosa is a protein described in any one of (i) and (ii) below:
   (i) a protein comprising the amino acid sequence represented by SEQ ID NO: 2; and
   (ii) a protein comprising an amino acid sequence obtained by substitution, deletion, insertion, or addition of one or more amino acids in the amino acid sequence represented by SEQ ID NO: 2, the protein being functionally equivalent to a protein including the amino acid sequence represented by SEQ ID NO: 2.
<3> The antibody or the functional fragment thereof according to <1>, which binds to a surface of Pseudomonas aeruginosa.
<4> The antibody or the functional fragment thereof according to <1>, which has an activity to suppress a cytotoxic activity of Pseudomonas aeruginosa toward a human airway epithelial cell.
<5> The antibody or the functional fragment thereof according to <1>, which has an antibacterial activity in a patient infected with Pseudomonas aeruginosa.
<6> The antibody or the functional fragment thereof according to <5>, which has an antibacterial activity against a Pseudomonas aeruginosa infection in a patient with a reduced neutrophil level.
<7> The antibody or the functional fragment thereof according to <1>, which binds to an epitope of an antibody produced by a hybridoma deposited under any one of accession numbers FERM BP-11055 and FERM BP-11056.
<8> A hybridoma deposited under any one of accession numbers FERM BP-11055 and FERM BP-11056.
<9> A vaccine composition for use in prevention or treatment of a disease associated with Pseudomonas aeruginosa, the vaccine composition comprising an antigen composition including any one of a protein antigen and a peptide antigen which are capable of inducing production of an antibody against a PA1698 protein derived from Pseudomonas aeruginosa, and optionally comprising at least one pharmaceutically acceptable carrier, diluent, and/or adjuvant.
<10> The vaccine composition according to <9>, wherein the PA1698 protein derived from Pseudomonas aeruginosa is a protein described in any one of (i) and (ii) below:
   (i) a protein comprising the amino acid sequence represented by SEQ ID NO: 2; and
   (ii) a protein comprising an amino acid sequence obtained by substitution, deletion, insertion, or addition of one or more amino acids in the amino acid sequence represented by SEQ ID NO: 2, the protein being functionally equivalent to a protein including the amino acid sequence represented by SEQ ID NO: 2.
<11> The vaccine composition according to <9>, wherein the disease associated with Pseudomonas aeruginosa is a systemic infectious disease caused by a Pseudomonas aeruginosa infection.
<12> A pharmaceutical composition for use in prevention or treatment of a disease associated with Pseudomonas aeruginosa, the pharmaceutical composition comprising the antibody or the functional fragment thereof according to any one of <1> to <7>, and optionally comprising at least one pharmaceutically acceptable carrier and/or diluent.
<13> The pharmaceutical composition according to <12>, wherein the disease associated with Pseudomonas aeruginosa is a systemic infectious disease caused by a Pseudomonas aeruginosa infection.
<14> A diagnostic agent for a Pseudomonas aeruginosa infection, comprising the antibody or the functional fragment thereof according to any one of <1> to <3>.
<15> A kit for detecting Pseudomonas aeruginosa, comprising the antibody or the functional fragment thereof according to any one of <1> to <3>.

### Detailed Description of the Preferred Embodiments

### [Antibody]

According to the present invention, provided is an antibody or a functional fragment thereof, which recognizes a PA1698 protein derived from Pseudomonas aeruginosa or a portion thereof. The amino acid sequence of the PA1698 protein of Pseudomonas aeruginosa, to which the antibody according to the present invention binds, is found to be extremely highly conservative among the strains regardless of serotypes and the like (Example 2) . Thus, the PA1698 protein of Pseudomonas aeruginosa, which is targeted by the antibody according to the present invention, is preferably a protein derived from a PA01 strain comprising the amino acid sequence described in SEQ ID NO: 2 or a protein comprising an amino acid sequence represented by SEQ ID NO: 2 in which one or more amino acids are substituted, deleted, inserted or added, the protein being functionally equivalent to a protein including the amino acid sequence represented by SEQ ID NO: 2. Generally, 1 to 2 amino acids are substituted, deleted, inserted or added in the amino acid sequence. Typically, 1 to 2 amino acids are substituted.

The antibody according to the present invention is preferably obtained by administering to an experimental animal (immunizing the experimental animal with) an antigen composition in such an amount that the antibody can be induced by immunizing the experimental animal, the antigen composition including a purified PA1698 protein as an antigen. The antibody can be used as a pure antibody by collecting blood from the heart or artery, separating antisera therefrom, and purifying the obtained antisera.

The antibody of the present invention includes: a polyclonal antibody or a monoclonal antibody, which is obtained by immunizing a mammal such as a mouse with the PA1698 protein serving as an antigen (including a monoclonal antibody produced by a hybridoma that produces a monoclonal antibody of the present invention); a chimeric antibody and a humanized antibody, which are prepared by using genetic recombination technique; and a human antibody prepared by using a human antibody-producing transgenic animal or the like. When the antibody according to the present invention is administered as a medicament to a human, the human antibody is desirable in terms of reducing side effects.

The "human antibody" is an antibody having all regions derived from a human. The human antibody according to the present invention can be prepared using a method well known to those skilled in the art (can be referred to, for example, Intern. Rev. Immunol, 1995, 13, 65-93, J. Mol. Biol, 1991, 222, 581-597, Japanese Unexamined Patent Application Publication No. Hei 10-146194, Japanese Unexamined Patent Application Publication No. Hei 10-155492, Japanese Patent No. 2938569, Japanese Unexamined Patent Application Publication No. Hei 11-206387, International Application Japanese-Phase Publication No. Hei 8-509612, International Application Japanese-Phase Publication No. Hei 11-505107, and the like).

The "humanized antibody" is an antibody prepared by transplanting only the gene sequence of the antigen-binding site (CDR; complementarity determining region) of a mouse antibody into a human antibody gene (CDR grafting). The humanized antibody according to the present invention can be prepared using a method well known to those skilled in the art (can be referred to, for example, EP 239400, WO 90/07861, and the like).

The "chimeric antibody" is an antibody prepared by ligating the variable region of an antibody of a certain species to the constant region of an antibody of a different species. Specifically, a mouse is immunized with an antigen, and an antibody variable region (V region) that binds to the antigen is cut out of the gene of the mouse monoclonal antibody. The obtained V region is then allowed to bind to an antibody constant region (C region) gene derived from human bone marrow. In this manner, the chimeric antibody can be prepared. The chimeric antibody according to the present invention can be prepared using a method well known to those skilled in the art (can be referred to, for example, Japanese Unexamined Patent Application Publication No. Hei 8-280387, US Patent No. 4816397, US Patent No. 4816567, US Patent No. 5807715, and the like).

The monoclonal antibody according to the present invention can be prepared using a method well known to those skilled in the art (can be referred to, for example, Antibodies A LABORATORY MANUAL, Ed Harlow and David Lane, Cold Spring Harbor Laboratory 1988; Experimental Manual for Monoclonal Antibody (1987) Kodansha, edited by Sakuji Toyama et al.; Monoclonal Antibody-Hybridoma and ELISA (1987) Kodansha, edited by Tatsuo Iwasaki et al; and the like) . The polyclonal antibody according to the present invention can also be prepared using a method well known to those skilled in the art.

The "functional fragment" according to the present invention means a part of an antibody (a partial fragment thereof), which specifically recognizes the protein according to the present invention. Specific examples thereof include Fab, Fab', F(ab')₂, a variable region fragment (Fv), disulfide-bonded Fv, a single chain antibody (scFv), and polymers thereof.

The antibody of the present invention can be used in treatment or diagnosis of a Pseudomonas aeruginosa infection, or as a reagent for research. Such an antibody includes an antibody which recognizes the PA1698 protein and which binds to a surface of Pseudomonas aeruginosa. When the Whole cell ELISA test described in Example 8 is carried out, a preferable antibody that binds to the surface of Pseudomonas aeruginosa has a significantly high absorbance in comparison with a control. In a case of a purified IgG fraction, the absorbance is preferably 0.3 or more, more preferably 0.5 or more, further preferably 0.8 or more, and still further preferably 1.0 or more (for example, 1.2 or more, 1.4 or more).

In the application form of the antibody of the present invention to a Pseudomonas aeruginosa infection, provided is an antibody or a functional fragment thereof, which has an antibacterial activity in a patient infected with Pseudomonas aeruginosa. In this form, a particularly preferable antibody includes an antibody or a functional fragment thereof against a protein comprising the amino acid sequence represented by SEQ ID NO: 2 or a amino acid sequence represented by SEQ ID NO: 2 including one or more conservative substitutions.

Once an antibody having the antibacterial activity against Pseudomonas aeruginosa is specified, those skilled in the art would be able to specify a peptide region that the antibody recognizes and to prepare various antibodies which binds to the region and shows the same activity. A preferable example of such an antibody includes an antibody which binds to an epitope of an antibody produced by a hybridoma deposited under any one of FERM BP-11055 and FERM BP-11056.

The patient infected with Pseudomonas aeruginosa may be, for example, a patient with a reduced neutrophil level due to administrations of various drugs, radiotherapy, or the like. The antibody of the present invention is advantageous in that the antibody is capable of exhibiting the effect on such a patient who is thus likely to develop serious infection. Meanwhile, Pseudomonas aeruginosa with which the patient is infected can be multidrug resistant Pseudomonas aeruginosa. The antibody of the present invention can also show the effectiveness on a patient infected with multidrug resistant Pseudomonas aeruginosa who cannot be treated with generally-used antibiotics.

Meanwhile, in chronic respiratory tract infections such as diffuse panbronchiolitis (DPB) and cystic fibrosis (CF), excessive secretion of mucus (mucin) from an airway epithelial cell causes airway obstruction, serving as a major factor of poor prognosis. Pseudomonas aeruginosa is a major causative bacterium of chronic respiratory tract infections. The antibody of the present invention is capable of suppressing a cytotoxic activity of Pseudomonas aeruginosa toward a human airway epithelial cell, and thus capable of exhibiting an effect on treatment of chronic respiratory tract infections, also. When the experiment described in Example 10 is carried out, a preferable antibody suppresses the cell death of human airway epithelial cell by 10% or more, more preferably 15% or more, and further preferably 20% or more. Examples of such an antibody include antibodies produced by hybridomas deposited under FERM BP-11055 and FERM BP-11056.

Additionally, according to the present invention, provided is a hybridoma producing the antibody of the present invention. A preferable hybridoma includes hybridomas deposited at the National Institute of Advanced Industrial Science and Technology, International Patent Organism Depositary (Central 6, 1-1-1, Higashi, Tsukuba, Ibaraki, postal code 305-8566, Japan) on October 31, 2008, under the accession numbers of FERM BP-11055 (1698-1) and FERM BP-11056 (1698-2).

### [Vaccine Composition]

The PA1698 protein derived from Pseudomonas aeruginosa can be used as a protein antigen. Accordingly, an antigen composition including such an antigen can be used as a vaccine for prevention or treatment of a disease associated with Pseudomonas aeruginosa. Thus, according to the present invention, provided is a vaccine composition comprising an antigen composition capable of inducing production of an antibody against the PA1698 protein derived from Pseudomonas aeruginosa. Here, the "antigen composition" may be a composition consisting of only the protein antigen as a constituent thereof, or may be a composition additionally including other components.

According to the present invention, a vaccine composition for use in prevention or treatment of a disease associated with Pseudomonas aeruginosa can be prepared, the vaccine composition comprising the above-described antigen composition, and optionally comprising at least one pharmaceutically acceptable carrier, diluent, and/or adjuvant.

The carrier used in the vaccine composition according to the present invention is selected on the basis of the mode and route of administration, and actual standard drug formulation. The carrier may be carrier proteins (for example, bovine serum albumin (BSA), ovalbumin (OVA), human serum albumin (HSA), hemocyanin derived from grand keyhole limpet (KLH: Keyhole limpet hemocyanin), and the like), solubilizers (for example, ethanol, polysorbate, Cremophor EL (registered trademark), and the like), isotonic agents, preservatives, antioxidants, excipients (for example, lactose, starch, crystalline cellulose, mannitol, maltose, calcium hydrogen phosphate, light anhydrous silicic acid, calcium carbonate, and the like), binders (for example, starch, polyvinylpyrrolidone, hydroxypropyl cellulose, ethyl cellulose, carboxymethyl cellulose, gum arabic, and the like), lubricants (for example, magnesium stearate, talc, hydrogenated oil, and the like), stabilizers (for example, lactose, mannitol, maltose, polysorbate, macrogol, polyoxyethylene hydrogenated castor oil, and the like), and the like. If necessary, glycerin, dimethylacetamide, 70% sodium lactate, a surfactant, a basic substance (for example, sodium hydroxide, ethylenediamine, ethanolamine, sodium bicarbonate, arginine, meglumine, trisaminomethane, or the like), or the like may be added.

As a specific example of the carrier protein, the peptide according to the present invention can be coupled to a known KLH solution (manufactured by Calbiotec Inc., 125 mg is dissolved per ml of a 50% glycerol solution), so as to enhance the antigenicity of the vaccine composition according to the present invention.

The diluent used in the vaccine composition according to the present invention is selected on the basis of the mode and route of administration, and actual standard drug formulation. Examples of the diluent include water or a saline, a phosphate-buffered saline, a bicarbonate solution, and the like.

The adjuvant used in the vaccine composition according to the present invention is selected on the basis of the mode and route of administration, and actual standard drug formulation. Examples of the adjuvant include cholera toxin, Escherichia coli heat-labile enterotoxin (LT), liposome, an immunostimulating complex (ISCOM: immunostimulating complex), and the like.

An administration may differ depending on the age, weight, sex, and general health state of an administration target at a risk of a Pseudomonas aeruginosa infection. The administration can be carried out by any administration route of oral administration and parenteral administration (for example, intravenous administration, intraarterial administration, and local administration). However, parenteral administration is preferable. The dosage form for oral administration and parenteral administration and the preparation method thereof are well known to those skilled in the art. The dosage form can be prepared according to a conventional process, for example, by mixing the antigen composition according to the present invention with the aforementioned pharmaceutically acceptable carrier or the like. Examples of the dosage form for oral administration include solid and liquid dosage forms, and specifically a solution, a tablet, a granule, a powder, and a capsule. Examples of the dosage form for parenteral administration include a solution, a suspension, an ointment, a cream, a suppository, an ophthalmic agent, nasal drops, and ear drops. In the case of oral administration, a flavoring agent and a coloring agent can also be added.

If the sustained release of the present preparation is desired, a biodegradable polymer (for example, poly-D,L-lactide-co-glycolide, polyglycolide, or the like) can be added as a bulk matrix (can be referred to, for example, US Patent No. 5,417,986, US Patent No. 4,675,381, and US Patent No. 4,450,150).

Appropriate pharmaceutical carriers, diluents, and the like, as well as pharmaceutically necessities for their use are described in Remington's Pharmaceutical Sciences.

The dose of the vaccine composition according to the present invention is determined by the present inventors depending on, for example, the type of vaccine antigen, whether or not the adjuvant is administered in combination with the present antigen, the type of adjuvant coadministered therewith, the mode and frequency of administration, and a desired effect (for example, a preventive or therapeutic effect). Generally, the dose of the vaccine composition of the present invention is 1 µg to 100 mg per administration for one adult. When the adjuvant is administered in combination with the present vaccine, the dose is generally 1 ng to 1 mg per administration for one adult. In accordance with the decision made by the present inventors, the administration is repeated when necessary. For example, following the initial administration, 3 booster administrations can be carried out per week. Alternatively, using the same formulations, a booster injection can be carried out on the 8th to 12th week after the first immunization and a second booster injection can be carried out on the 16th to 20th week after the first immunization.

### [Use of Antibody and Pharmaceutical Composition] Disease Associated with Pseudomonas aeruginosa

Pseudomonas aeruginosa is a pathogen of opportunistic infections which cause fatal consequences with reductions in the resistance of hosts. Moreover, since being resistant to antibiotics, Pseudomonas aeruginosa is a major causative bacterium of hospital infections. As shown in Examples described later, it has been confirmed that the antibody of the present invention actually has a protective effect against infections on a Pseudomonas aeruginosa infection-susceptible murine model with macrophage functions reduced by mucin administration (Example 11), and that the antibody according to the present invention actually has a protective effect against infections on a Pseudomonas aeruginosa infection-susceptible murine model with a neutrophil level reduced by cyclophosphamide monohydrate administration (Example 12). Furthermore, a reason why the antibody according to the present invention has the protective effect against infections includes the ability to suppress a cytotoxic activity of Pseudomonas aeruginosa (Example 10). Therefore, the antibody according to the present invention is useful in prevention or treatment of a disease associated with Pseudomonas aeruginosa.

Examples of the disease associated with Pseudomonas aeruginosa include systemic infectious diseases, caused by a Pseudomonas aeruginosa infection including a multidrug resistant Pseudomonas aeruginosa infection, for example, septicemia, meningitis, and endocarditis. Other examples include: otitis media and sinusitis in the otolaryngologic field; pneumonia, chronic respiratory tract infection, and catheter infection in the pulmonary field; postoperative peritonitis and postoperative infection in a biliary duct or the like in the surgical field; abscess of eyelid, abscess of nasolacrimal duct, conjunctivitis, corneal ulcer, corneal abscess, panophthalmitis, and orbital infection in the ophthalmological field; and urinary tract infections including complicated urinary tract infection, catheter infection, and abscess around the anus in the urologic field. Besides, the examples include burns including a serious burn and a burn of the respiratory tract, decubital infection, and cystic fibrosis.

According to the present invention, provided is a prevention method or treatment method of the disease associated with Pseudomonas aeruginosa, the method comprising a step of administering a preventively or therapeutically effective amount of the antibody according to the present invention to mammals including a human.

### Diagnostic Agent for Pseudomonas aeruginosa Infection

As shown in Examples described later, it has been confirmed that the antibody according to the present invention binds to Pseudomonas aeruginosa (Example 8). These results suggest that the antibody according to the present invention be capable of detecting the presence of Pseudomonas aeruginosa. Thus, the antibody according to the present invention can be used as a diagnostic agent for a Pseudomonas aeruginosa infection.

Moreover, according to the present invention, provided is a diagnosis method for a Pseudomonas aeruginosa infection using the antibody according to the present invention.

The diagnosis method according to the present invention can be carried out by collecting a biological sample such as sputum, a lung lavage fluid, pus, a tear, blood, or urine from mammals including a human at a risk of a Pseudomonas aeruginosa infection, subsequently bringing the collected sample into contact with the antibody according to the present invention, and determining whether or not an antigen-antibody reaction occurs.

### Diagnostic Agent Kit for Pseudomonas aeruginosa Infection

According to the present invention, provided is a kit for detecting the presence of Pseudomonas aeruginosa, the kit comprising at least the antibody according to the present invention. The antibody that the detection kit according to the present invention comprises may be labeled. This detection kit is capable of detecting the presence of Pseudomonas aeruginosa by utilizing the antigen-antibody reaction.

The detection kit according to the present invention can further comprise various reagents for carrying out the antigen-antibody reaction, for example, a secondary antibody, a chromogenic reagent, a buffer, instructions, and/or an instrument, etc. which are used in an ELISA method or the like, if desired.

### Pharmaceutical Composition

A pharmaceutical composition or an agent according to the present invention may be used in the form of a composition which uses the antibody according to the present invention as an active ingredient, and preferably which contains a purified antibody composition and another component, for example, a saline, an aqueous glucose solution or a phosphate buffer.

The pharmaceutical composition according to the present invention may be formulated in a liquid or freeze-dried form as necessary, and may optionally comprise a pharmaceutically acceptable carrier, for example, a stabilizer, a preservative, and an isotonic agent.

Examples of the pharmaceutically acceptable carrier can include: mannitol, lactose, saccharose, and human albumin for a freeze-dried preparation; and saline, water for injection, a phosphate buffer, and aluminium hydroxide for a liquid preparation. However, the examples are not limited to these.

An administration may differ depending on the age, weight, sex, and general health state of an administration target. The administration can be carried out by any administration route of oral administration and parenteral administration (for example, intravenous administration, intraarterial administration, and local administration). However, parenteral administration is preferable.

The dose of the pharmaceutical composition varies depending on the age, weight, sex, and general health state of a patient, the severity of a Pseudomonas aeruginosa infection and components of an antibody composition to be administered. The dose of the antibody composition according to the present invention is generally 0.1 to 1000 mg, and preferably 1 to 100 mg, per kg body weight for an adult per day for intravenous injection.

The pharmaceutical composition according to the present invention is preferably administered in advance to a patient at a risk of a Pseudomonas aeruginosa infection.

When the pharmaceutical composition is prepared as a diagnostic agent, this diagnostic agent can be obtained in any dosage form by adopting any means suitable for its purpose. For example, ascites, a culture solution containing an antibody of interest, or a purified antibody is measured for the antibody titer and appropriately diluted with PBS (phosphate buffer containing a saline) or the like; thereafter, a preservative such as 0.1% sodium azide is added thereto. Alternatively, the antibody of the present invention adsorbed to latex or the like is determined for the antibody titer and appropriately diluted, and a preservative is added thereto for use. The antibody of the present invention bound to latex particles as described above is one of preferable dosage forms as a diagnostic agent. As the latex in this case, appropriate resin materials, for example, latex such as polystyrene, polyvinyl toluene, or polybutadiene, are suitable.

### Examples

Hereinafter, the present invention will be described in line with Examples for promoting the understanding of the present invention. However, the present invention is not limited to these Examples.

### <Example 1: GeneChip^{R} Analysis>

GeneChip^{R} expression analysis system (manufactured by Affymetrix, Inc., Genechip^{R} P. aeruginosa genome array) was used as an approach for identifying genes that are expressed in a medium supplemented with human sera. Shake culture was carried out using a Pseudomonas aeruginosa PAO1 strain under two different culture conditions, that is, in Luria-Bertani (LB) media (manufactured by Nacalai Tesque, Inc.) supplemented with 0% and 50% human sera (the final composition of the LB media was equal among them) at 37°C until the absorbance at 595 nm reached 1.0. Using RNeasy Protect Bacteria Mini kit (manufactured by QIAGEN GmbH), total RNA was extracted according to the method described in documents included therewith and quantified using 2100 bioanalyzer (manufactured by Agilent Technologies, Inc.). Then, experiments were carried out according to the method described in documents included with GeneChip^{R}. Gene expression data was analyzed using Microarray Suite 5.0 (manufactured by Affymetrix, Inc.), and signal and detection were calculated. At this time, correction was carried out such that the average value of the signals from all probe sets was 1000. Two independent experiments were carried out.

As a result, PA4761 (DnaK or HSP70), which is a house keeping protein, was determined, under any of the culture conditions regardless of the presence or absence of supplemented sera, to be "Present" that indicates that a transcription product was detected. It was thus shown that the gene was expressed. Moreover, PA2018 (MexY) (J. Bacteriology, 2005, 187, 5341-5346), which is an inner membrane-spanning protein that associates with PA5158 (OpmG) and PA2019 (MexX) so as to constitute drug efflux pumps, and which is induced by ribosome inhibitors such as tetracycline or aminoglycoside antibiotics, was determined, under these conditions free of these drugs, to be "Absent". It was thus shown that the gene was not expressed. By contrast, a PA1698 gene was determined to be "Present" under any of the conditions regardless of the presence or absence of supplemented sera.

Thus, the PA1698 gene was certainly expressed, and the possibility that its gene product PA1698 protein is constantly present was suggested.

### <Example 2: Analysis of PA1698 gene in Clinical Isolates>

Bacterial strains used were 93 Pseudomonas aeruginosa strains (stored in Yokohama Research Lab., Meiji Seika Kaisha, Ltd.) isolated from various types of clinical materials in clinical facilities across Japan, and the strains were subjected to tests. These strains were derived from blood, urine, sputum, pus, pharyngeal mucus, and the like, and their serotypes include groups A, B, E, G, I, M, etc., based on serological classification according to the decision of the serotyping committee sponsored by Japan Pseudomonas aeruginosa Society (1975) .

### (1) Preparation of Genomic DNA

Each of 93 clinical isolates of Pseudomonas aeruginosa was cultured overnight at 37°C in a Mueller-Hinton medium (manufactured by Becton, Dickinson and Company), and collected by low-speed centrifugation. Using DNeasy Tissue kit (manufactured by QIAGEN GmbH), genomic DNA was prepared from the obtained bacterial cells according to the method described in documents included therewith.

### (2) Amplification of DNA Fragment by PCR Method

Using the prepared genomic DNA as a template, a region containing the PA1698 gene was amplified by PCR. Specifically, a primer set (SEQ ID NO: 3 and SEQ ID NO: 4) for specifically amplifying the PA1698 gene was designed based on the genomic sequence of the Pseudomonas aeruginosa PAO1 (NCBI database accession No: NC_002516). Using GeneAmp PCR System 9700 (manufactured by Applied Biosystems Inc.), PCR was carried out with Takara ExTaq (manufactured by Takara Bio Inc.) according to instructions included therewith. The DNA fragment thus amplified by PCR was confirmed by agarose gel electrophoresis to have the size of interest (1128 base pairs).

### (3) Analysis of Polynucleotide Sequence Using DNA Sequencer

The PCR product was purified using MultiScreen PCR plate (manufactured by Millipore Corporation) and then subjected to a sequencing reaction. Primers (SEQ ID NO: 5 to SEQ ID NO: 7) capable of sequencing each PCR product were designed based on the genomic sequence of the PAO1 strain (NC_002516). BigDye Terminator v1.1 Cycle Sequencing kit (manufactured by Applied Biosystems Inc.) was used in the sequencing reaction. The sequencing reaction was carried out using GeneAmp PCR System 9700 (manufactured by Applied Biosystems Inc.) according to instructions included therewith. The sequencing reaction product was purified using MultiScreen-HV plate (manufactured by Millipore Corporation) filled with Sephadex G-50 Fine DNA Grade (manufactured by Amersham Biosciences AB) which had been swollen with water in advance. Then, the polynucleotide sequence was analyzed using Applied Biosystems 3730 DNA Analyzer (manufactured by Applied Biosystems Inc.).

The polynucleotide sequences of the clinical isolates revealed by the aforementioned analysis were converted to polypeptide sequences. These polypeptide sequences were compared with those from the PAO1 strain. As a result of study, 15 mutations were observed in the full-length sequence of the PA1698 protein (Table 1).

However, it was confirmed that approximately 70% of the clinical isolates had the amino acid sequence completely conserved, and that even remaining approximately 30% of the clinical isolates had only 1 or 2 amino acid mutations. Accordingly, all Pseudomonas aeruginosa had conserved regions. This suggested that the Pseudomonas aeruginosa PA1698 protein is useful as a "Pseudomonas aeruginosa common antigen".

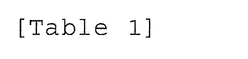

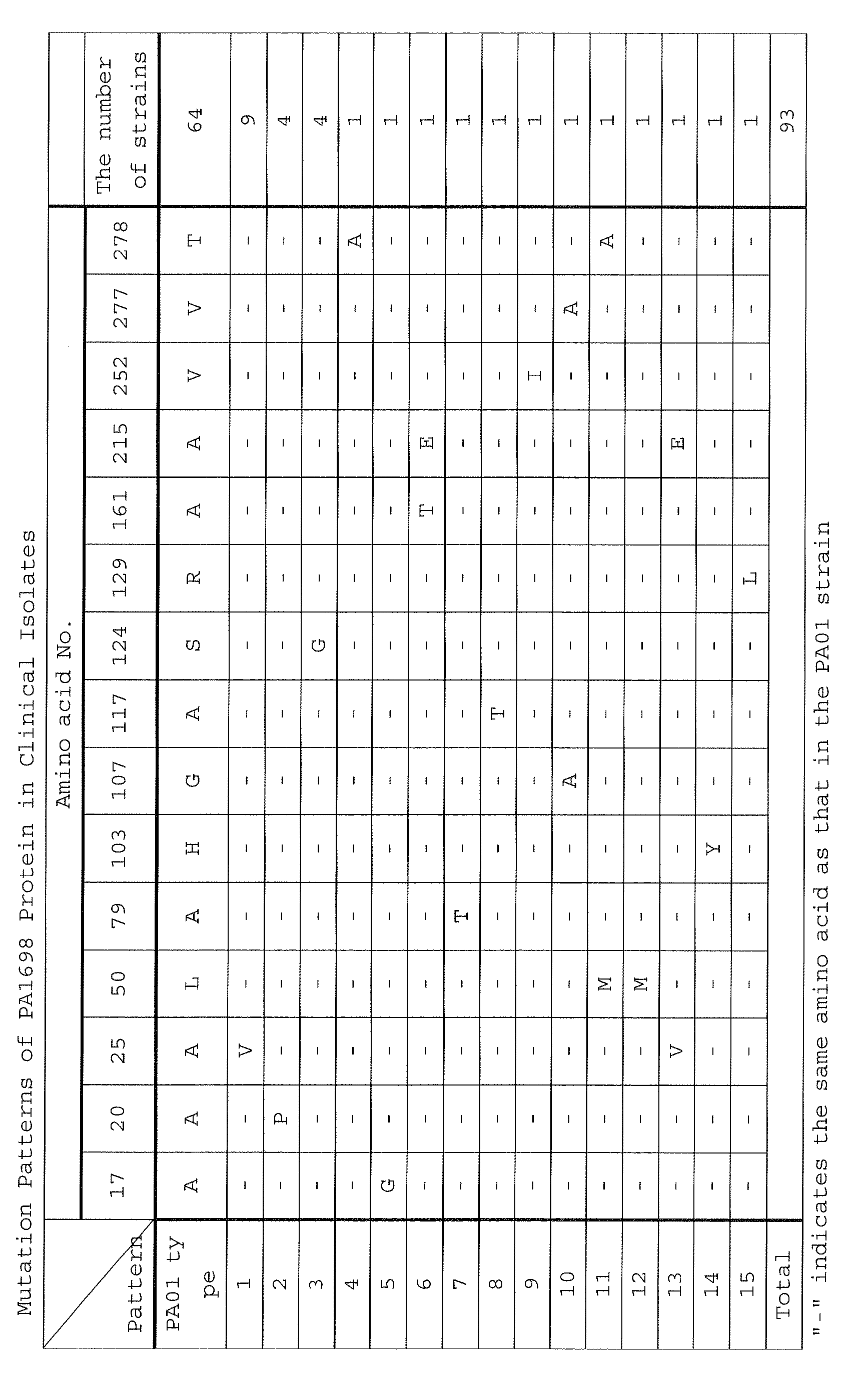

### <Example 3: Cloning of PA1698 Gene DNA Fragment>

A DNA fragment containing 867 bases as a full-length amino acid coding region of the Pseudomonas aeruginosa PA1698 gene (SEQ ID NO: 1) was cloned using a vector pIVEX2.4d (Roche Diagnostics K. K.) and incorporated into an expression vector pET15b (Novagen Inc.) by the following method.

The DNA fragment to be cloned was amplified from the genomic DNA of the Pseudomonas aeruginosa PAO1 strain by PCR (DNA Thermal Cycler 480; manufactured by Perkin-Elmer Inc.). Pyrobest (manufactured by Takara Shuzo Co., Ltd.) was used as a DNA polymerase. Five percent of dimethyl sulfoxide was added to a reaction solution. Primers (SEQ ID NO: 8 and SEQ ID NO: 9) containing bases used for adding restriction sites NotI (GCGGCCGC) and BamHI (GGATCC) were used as PCR primers.

The temperature conditions set in PCR was at 94°C for 2 minutes and 25 cycles each consisting of heating at 94°C for 30 seconds, at 60°C for 1 minute, and then at 72°C for 2 minutes. The PCR product was purified using MinElute PCR Purification Kit (manufactured by Qiagen GmbH), and then digested with restriction enzymes NotI (manufactured by New England Biolabs Inc.) and BamHI (manufactured by Toyobo Co., Ltd.). Moreover, pIVEX2.4d was digested with NotI and BamHI. The DNA fragments thus obtained by the digestion were separated by agarose gel electrophoresis, and extracted and purified using QIAquick Gel Extraction Kit (Manufactured by Qiagen GmbH). The PCR product and pIVEX2.4dthus digested with NotI-BamHI were ligated using T4 DNA ligase (Ligation High, manufactured by Toyobo Co., Lid.), and transformed into Escherichia coli DH5α strain (Competent High DH5α, manufactured by Toyobo Co., Ltd.). A pIVEX2.4d plasmid (pIVEX-PA1698-1) having the PA1698 gene fragment incorporated therein was purified using QIAprep Spin Miniprep Kit (Manufactured by Qiagen GmbH). Then, a cycle sequencing reaction was carried out using BigDye Terminator v1.1 Cycle Sequencing Kit (manufactured by Applied Biosystems Inc.), and the base sequence of the inserted portion was confirmed (3730 DNA Analyzer, manufactured by Applied Biosystems Inc./HITACHI Ltd.).

Next, with pIVEX-PA1698-1 used as a template, a fragment containing the insertion portion was amplified using a PCR primer (SEQ ID NO: 10) and a PCR primer (SEQ ID NO: 11). Here, the PCR primer (SEQ ID NO: 10) had a start codon (ATG) containing a base for adding a restriction site NdeI (CATATG) while the PCR primer (SEQ ID NO: 11) paired with a T7 terminator portion positioned downstream of the insertion portion. The fragment was digested with restriction enzymes NdeI (manufactured by New England Biolabs Inc.) and BamHI having a restriction site immediately after a stop codon. Moreover, pET15b was digested with NdeI and BamHI. After separated and purified, these digested DNA fragments were ligated to be transformed into Escherichia coli. A pET15b plasmid (pET-PA1698-1) having the PA1698 gene fragment incorporated therein was collected, and the base sequence of the insertion portion was confirmed.

### <Example 4: Expression and Purification of PA1698 recombinant Protein>

A pET vector expression system (manufactured by Novagen Inc.) having a T7 promoter and an Escherichia coli BL21 (DE3) strain with a T7 RNA polymerase gene incorporated therein was used for expression of a recombinant protein. The Escherichia coli expression vector pET-PA1698-1 is a plasmid encoding a PA1698 protein in which His-tag (6 consecutive histidines) has been fused downstream of the T7 promoter (see Example 3). The BL21 (DE3) strain was treated with calcium chloride (see Molecular Cloning 2nd ed. Sambrook et al. (1989)) and transformed with the pET-PA1698-1. The transformant was cultured overnight in an LB medium containing 50 µg/ml ampicillin, and diluted 200-fold in a fresh medium and suspended. After 4 hours of culturing at 37°C, the expression was induced by addition of IPTG at a final concentration of 0.5 mM, and the culturing was continued for additional 3 hours. The cells were collected by centrifugation, and frozen at -20°C. The cells were dissolved in a protein extraction reagent of B-PER Bacterial Protein Extraction Reagent (manufactured by Pierce Biotechnology Inc.). An insoluble fraction containing the expressed protein was collected and treated with lysozyme (egg-white lysozyme, manufactured by Seikagaku Corporation) at a final concentration of 100 µg/ml, followed by washing with a Dulbecco's phosphate-buffered saline (PBS) supplemented with 1% Triton X-100.

Ni chelate chromatography utilizing the His-tag was used for protein purification. The insoluble protein thus expressed and prepared was solubilized with a dissolution buffer (PBS supplemented with 8 M urea, 5 mM imidazole, 200 mM NaCl, and 0.05% NP-40). The dissolved protein was bound to Ni-NTA Agarose (Manufactured by Qiagen GmbH), and washed with 40 volumes of a dissolution buffer. The protein was further washed with 40 volumes of a wash buffer (a dissolution buffer from which NP-40 was excluded). Then, the His-tag-attached protein was eluted with an elution buffer (PBS supplemented with 8 M urea, 300 mM imidazole, and 200 mM NaCl), and collected. As a result, 2.3 mg of the protein was finally collected from 230 ml of the Escherichia coli culture.

### <Example 5: Immunization with Antigen and Preparation of Antisera>

An inactivated Pseudomonas aeruginosa obtained in the following manner was used here. A Pseudomonas aeruginosa PA103 strain (ATCC29260) was cultured overnight at 37°C on a Mueller-Hinton agar medium. Several colonies thereof were suspended in an LB medium, then shake-cultured overnight at 37°C, washed with PBS, and resuspended. Subsequently, inactivation treatment was carried out for 24 hours or longer by addition of 1% formalin.

For use in immunization, the PA1698 recombinant protein was dissolved in an 8 M urea solution, resulting in a concentration of 100 µg/ml.

In an immunization method, a male BN rat (purchased from Charles River Laboratories Japan, Inc.) was subcutaneously or intramuscularly immunized with 6 shots in total, in combination with a Freund complete adjuvant only in the first shot, and in combination with an incomplete adjuvant in the subsequent shots, at 2-week intervals. For the immunization, 20 µg of each of the formalin-inactivated bacteria and the PA1698 recombinant protein was administered per animal. One week after the final immunization, whole blood was collected from carotid artery or abdominal aorta. The blood was left at room temperature for one hour, and then centrifuged (1500G, 20 minutes), so as to collect approximately 5 ml/rat of a supernatant as antisera.

### <Example 6: Purification of IgG Fractions from Antisera and Ascites>

IgG fractions from the rat antisera and ascites were purified by using the method of, for example, Harlow & Lane, 288-318, Chapter 8, Antibodies, A Laboratory Manual, Cold Spring Harbor (1988) or the like. The rat antisera or ascites obtained by proliferating a rat-mouse hybridoma in the mouse abdominal cavity were centrifuged at 10,000 × g for 20 minutes. An insoluble matter was removed therefrom, and a supernatant was obtained. Two volumes of 60 mM sodium acetate (pH 4.0) was added, and then the pH was adjusted to 4.8 with 1 N hydrochloric acid. 0.06 volumes of caprylic acid relative to the antisera or ascites sample was gradually added at room temperature, and the mixture was stirred for 30 minutes, so as to produce an insoluble matter. Precipitates were removed by centrifugation at 13, 000 × g for 10 minutes. The resulting solution was then passed through a 0.45 µm filter. The obtained sample was concentrated using Amicon Ultra-15 (Millipore Corporation), and the resultant was finally exchanged with a PBS (-) solution, so as to obtain a final sample. By this method, 102 mg, 14 mg, and 3 mg of proteins were collected as the IgG fraction by purification from 40 ml of the rat antisera or 75 mL and 35 mL of the mouse ascites containing rat MAb produced from hybridomas under the accession numbers FERM BP-11055 and FERM BP-11056 at the National Institute of Advanced Industrial Science and Technology, International Patent Organism Depositary. The proteins were quantified according to DC Protein Assay (manufactured by Bio-Rad Laboratories, Inc.) based on the Lowry method, and the IgG purity was evaluated by SDS-PAGE.

### <Example 7: ELISA Test>

In order to detect by the ELISA method an antibody that binds to the PA1698 recombinant protein, the PA1698 recombinant protein was dissolved in PBS supplemented with 8 M urea. 0.5 µg of the protein was placed per well of a 96-well nickel plate (HIS-Select High Sensitivity (HS) Nickel Coated Plates, manufactured by Sigma-Aldrich Co.). The protein was bound to the plate at room temperature. The plate was washed with a wash buffer (PBS supplemented with 0.05% Tween 20, 5 mM imidazole and 500 mM NaCl), and blocked with a blocking buffer (a wash buffer supplemented with 0.5% gelatin). Then, a sample including the antibody obtained in Example 5 or 6 was placed in the well and allowed for reaction at room temperature. The plate was washed with a wash buffer thereafter. A secondary antibody (peroxidase-labeled goat anti-rat IgG antibody, 10000-fold diluted, manufactured by Sigma-Aldrich Co.) was placed therein and allowed for reaction. The plate was washed thereafter. A chromogenic substrate (TMB Microwell Peroxidase Substrate System, manufactured by KPL Inc.) was added for reaction, and then the enzyme reaction was terminated with 1 M phosphoric acid. The absorbance at 450 nm was measured.

As a result, the absorbance of the PA1698 recombinant protein immunized-rat antisera (10,000-fold diluted) sample was 0.490, whereas the absorbance of negative control sera before immunization (10,000-fold diluted) was 0.061. This indicates that the antibody which binds to the PA1698 recombinant protein as an immunogen is contained in the PA1698 recombinant protein immunized-rat antisera.

### <Example 8: Whole Cell ELISA Test>

For Whole cell ELISA, 100 µL of a bacterial solution of the PA103 strain cultured overnight in an LB medium was dispensed into each well of a 96-well ELISA plate (MaxiSorp Type, manufactured by Nunc), followed by immobilization at 4°C for one hour. Then, the plate was washed with a wash buffer (TBS containing 0.05% Tween 20), and blocked with a blocking buffer (TBS containing 2% bovine serum albumin). Thereafter, the purified IgG fraction or the PA1698 recombinant protein immunized-rat antisera obtained in Examples 5 and 6, which had been diluted with PBS, were added thereto as a primary antibody sample and allowed for reaction at 37°C for one hour. After washing, a secondary antibody (peroxidase-labeled goat anti-rat IgG antibody, 5000-fold diluted, manufactured by Sigma-Aldrich Co.) was added for reaction at room temperature for one hour, followed by washing. A chromogenic substrate (TMB Microwell Peroxidase substrate System, manufactured by KPL Inc.) was added for reaction in the dark, and then the enzyme reaction was terminated with a 1 M phosphoric acid solution. The absorbance at 450 nm was measured.

As a result, the absorbance of the PA1698 recombinant protein immunized-rat antisera was 1.058, whereas the absorbance of negative control sera before immunization was 0.729. This result indicates that the antibody (IgG) which recognizes the PA1698 protein existing on the Pseudomonas aeruginosa cell surface or in the culture supernatant is contained in the PA1698 recombinant protein immunized-rat antisera.

Meanwhile, with regard to the anti-PA1698 IgG that is the purified IgG fraction obtained from the PA1698 recombinant protein immunized-rat antisera, the absorbance of a negative control IgG fraction (50 µg/well) purified from control rat sera obtained by administering only adjuvant was 0.147, whereas that of the anti-PA1698 IgG (50 µg/well) was 0.460. This indicates that the antibody (IgG) which recognizes Pseudomonas aeruginosa is contained in the IgG fraction.

### <Example 9: Preparation of Monoclonal Antibody (MAb)>

One week after the final immunization with the PA1698 recombinant protein in Example 5, the spleen was aseptically extracted from the rat under anesthesia. The obtained spleen was washed with an RPMI-1640 medium (manufactured by Gibco Cop.). Then, the spleen was inserted between slide glasses and crushed, so as to obtain a splenic cell test sample in the form of fine small pieces. The obtained splenic cells were washed by centrifugation at 1000 rpm for 5 minutes using an RPMI-1640 medium. Meanwhile, myeloma cells (P3X63Ag8U1 cells) were cultured in advance under conditions of 5% CO₂, relative humidity of 100% and 37°C in an RPMI-1640 medium containing 10% FCS (fetal bovine serum). The myeloma cells during the exponential growth phase were washed by centrifugation using an RPMI-1640 medium. The aforementioned splenic cells and the myeloma cells were mixed with each other, such that the ratio of the splenic cells to the myeloma cells was 4:1. The mixture cells were centrifuged at 1000 rpm for 5 minutes. The supernatant was discarded, and the cells were sufficiently loosened. To a centrifuge tube containing the cells, 1 mL of a solution consisting of 2 g of polyethylene glycol (M.W.1000, manufactured by Wako Pure Chemical Industries, Ltd.), 2 mL of an RPMI-1640 medium and 0.2 mL of DMSO (manufactured by Nacalai Tesque, Inc.) was gently added. The centrifuge tube was slowly rotated to mix the cells. One minute later, while the centrifuge tube was slowly rotated, 15 mL of an RPMI-1640 medium was added thereto over three minutes. The cells were centrifuged at 1000 rpm for 5 minutes. Then, the supernatant was discarded, and the cells were sufficiently loosened. Thereafter, the cell concentration was adjusted to 1.6×10⁶ cells/mL in terms of the splenic cells using a HAT medium (manufactured by Gibco Corp.). The resulting cells were dispensed at a concentration of 0.2 mL/well into a 96-well microplate (manufactured by Sumitomo Bakelite Co., Ltd.). The cells were cultured under conditions of 5% CO₂, relative humidity of 100% and 37°C for approximately 1 to 2 weeks. After that, hybridomas grown in the wells were observed under a microscope.

### (1) Screening of Antibody of Interest

An antibody which binds to the PA1698 recombinant protein was detected by the ELISA method described in Example 7. Moreover, an antibody which binds to Pseudomonas aeruginosa was detected by the whole cell ELISA method described in Example 8.

### (2) Cloning of Cells Producing Antibody of Interest

As a result of the screening, the concentration of the hybridomas that were determined to produce the antibody of interest was adjusted to 5 hybridomas/0.2 mL or 20 hybridomas/0.2 mL using a 10% FCS/HT (manufactured by Gibco Corp.) medium containing 5% BM-Condimed H1 Hybridoma Cloning Supplement (manufactured by Roche Diagnostics K. K.). The hybridomas were dispensed at a concentration of 0.2 mL/well. One to two weeks later, the growth of clones was observed under a microscope. The clones were analyzed by the method described in the section of screening, so as to select clones producing the antibody of interest. Again, the concentration of the hybridomas was adjusted to one hybridoma/0.2 mL or two hybridomas/0.2 mL using a 10% FCS/HT (manufactured by Gibco Corp.) medium containing 5% BM-Condimed H1 Hybridoma Cloning Supplement by the above-described method. Such hybridomas were dispensed at a concentration of 0.2 mL/well of a 96-well microplate. One to two weeks later, the analysis was carried out by the method described in the section of screening to select monoclones producing the antibody of interest. Accordingly, obtained were the hybridomas under the accession numbers of FERM BP-11055 and FERM BP-11056 at the National Institute of Advanced Industrial Science and Technology, International Patent Organism Depositary.

### (3) In Vitro Culture of Cells and Production of MAb

The clones of interest sufficiently proliferated in the 96-well microplate were scaled up gradually in a 48-well plate, a 12-well plate, a 50-mL flask, and a 250-mL flask, and cultured in a 10% FCS-RPMI medium. The cells obtained in this manner had MAb produced in the culture supernatant thereof, the MAb being detected by the ELISA method described in Example 7.

As a result, the absorbance in the ELISA for detecting the binding to the well, on which the PA1698 recombinant protein was adsorbed, was 0.056 in the negative control 10% FCS-RPMI medium. Meanwhile, the absorbance of the culture supernatant of the hybridoma under the accession number of FERM BP-11055 was 0.828, whereas the absorbance of the culture supernatant of the hybridoma under the accession number of FERM BP-11056 was 0.811. From the result described above, it was demonstrated the MAb which bound to the PA1698 recombinant protein was produced.

### (4) In Vivo Cell Propagation in Ascites and Production of MAb

Each of the hybridomas under the accession numbers of FERM BP-11055 and FERM BP-11056 at the National Institute of Advanced Industrial Science and Technology, International Patent Organism Depositary, was intraperitoneally administered in a BALB/c-nu/nu mouse (purchased from Charles River Laboratories Japan, Inc.) at a concentration of 1×10⁷/mouse. One to two weeks later, the ascites was collected. MAb contained in the ascites was purified by the method described in Example 6. The obtained purified IgG fractions were designated as anti-1698-1 IgG (MAb) and anti-1698-2 IgG (MAb). The heavy chain and light chain of the IgG subclass of this rat MAb were determined by monoclonal antibody isotyping kit (RMT1, manufactured by Dainippon Pharmaceutical Co., Ltd.). As a result, it was determined that the heavy chains were IgG2a and IgG2a, respectively, and that all the light chains were κ.

The binding to the Pseudomonas aeruginosa surface of the MAb purified from the mouse ascites by the method descried in Example 6 was confirmed by whole cell ELISA described in Example 8.

Result for anti-1698-1 IgG (MAb) : the absorbance of the anti-1698-1 IgG (MAb), i.e. , the IgG fraction obtained by purifying the mouse ascites to which the hybridoma under the accession number of FERM BP-11055 had been administered, was 0.871. Meanwhile, the absorbance of a negative control IgG fraction (50 µg/well) purified from control rat sera obtained by administering only adjuvant was 0.084. This result demonstrated that the antibody (IgG) which recognizes Pseudomonas aeruginosa was contained in the IgG fraction.

Result for anti-1698-2 IgG (MAb) : the absorbance of the anti-1698-2 IgG (MAb), i.e., the IgG fraction obtained by purifying the mouse ascites to which the hybridoma under the accession number of FERM BP-11056 had been administered, was 1.415. Meanwhile, the absorbance of a negative control IgG fraction (50 µg/well) purified from control rat sera obtained by administering only adjuvant was 0.084. This result demonstrated that the antibody (IgG) which recognizes Pseudomonas aeruginosa was contained in the IgG fraction.

### <Example 10: Effect of PA1698 Antibodies to Defend Against Cytotoxic Activity of Pseudomonas aeruginosa PA103 Strain toward Human Airway Epithelial Cell (Beas 2B Cell)>

Beas 2B cells were dispensed at a cell concentration of 5×10⁴/well into a 96-well culture plate, and cultured in a 5% CO₂ incubator at 37°C for 2 days. After the culturing, the cells were washed with PBS (-) twice, and 50 µL of a DMEM/F12 medium supplemented with 16% BSA (after the dissolution, neutralized with KOH) was added. 25 µL of each of a Pseudomonas aeruginosa PA103 strain at 5×10⁸ cfu/mL and the anti-1698-1 IgG (MAb), the anti-1698-2 IgG (MAb), or the anti-PA1698 IgG, i.e., the purified IgG fraction obtained from the PA1698 recombinant protein immunized-rat antisera, which had been diluted with PBS, was added thereto and cultured in the 5% CO₂ incubator at 37°C for 4 hours. Four hours later, the amount of LDH (lactate dehydrogenase) in the culture supernatant was measured as an index of cell death. The amount of cell death was calculated as follows. The amount of cell death is set 100% if the amount of LDH measured is equivalent to that obtained when the cells are solubilized with 0.2% Triton X-100. Meanwhile, the amount of cell death is set 0% if the amount of LDH measured is equivalent to that obtained when the Pseudomonas aeruginosa PA103 strain is not added.

As a result, the cell death was suppressed by 16.3% by the addition (2.5 mg/mL) of the anti-PA1698 IgG, i.e. , the purified IgG fraction obtained from the PA1698 recombinant protein immunized-rat antisera. Moreover, the anti-1698-1 IgG (MAb) (0.625 mg/mL), i.e., the IgG fraction obtained by purifying the mouse ascites to which the hybridoma under the accession number of FERM BP-11055 had been administered, suppressed the cell death by 18.9%. Furthermore, the anti-1698-2 IgG (MAb) (0.625 mg/mL), i.e., the IgG fraction obtained by purifying the mouse ascites to which the hybridoma under the accession number of FERM BP-11056 had been administered, suppressed the cell death by 22.5%.

### <Example 11: Ability of PA1698 Recombinant Protein Immunized-Rat Antisera to Defend against PA103 Strain Systemic Infection in Normal Mice>

In evaluation with systemically infected models of normal mice, the PA103 strain suspended in 500 µl of a saline containing 5% mucin was intraperitoneally inoculated to 4-week-old CD-1 male mice (purchased from Charles River Laboratories Japan, Inc.) at a dose of 1.7×10⁵ cfu/mouse (34LD₅₀). Immediately thereafter, the serum sample 2.5-fold diluted with a saline was administered at a dose of 10 ml/kg from the caudal vein. The protective activity against the infection was assessed based on survival after 7 days.

As a result, all of 7 mice died in a group to which negative control rat sham sera obtained by administering only adjuvant had been administered. By contrast, all mice survived in a group to which formalin-inactivated PA103 strain immunized-rat antisera obtained in Example 5 had been administered. Thus, the protective activity against the infection was confirmed. Under this condition, 5 out of 7 mice survived in a group to which the PA1698 recombinant protein immunized-rat antisera obtained in Example 5 had been administered. Thus, the protective activity against the infection was confirmed.

### <Example 12: Ability of PA1698 Recombinant Protein Immunized-Rat Antisera to Defend against PA103 Strain Systemic Infection in Neutropenic Mice>

In evaluation with systemically infected models of neutropenic mice, 12.5 mg/mL (saline) of cyclophosphamide (hereinafter referred to as CY. manufactured by Sigma-Aldrich Co.) was prepared and intraperitoneally administered to 4-week-old CD-1 male mice at three doses in total on day-5, -2, and 0 each at 125 mg/kg, so as to reduce the neutrophil level in the peripheral blood. Then, the PA103 strain suspended in 250 µl of a saline was intraperitoneally inoculated at a dose of 9.5×10⁴ cfu/mouse (70LD₅₀). Immediately thereafter, the sample was administered at a dose of 10 mL/kg from the caudal vein. The protective activity against the infection was assessed based on survival after 7 days.

As a result, all of 7 mice died in a group to which negative control rat sham sera obtained by administering only adjuvant had been administered. By contrast, 6 out of 7 mice survived in a group to which formalin-inactivated PA103 strain immunized-rat antisera obtained in Example 5 had been administered. Thus, the protective activity against the infection was confirmed. Under this condition, 4 out of 7 mice survived in a group to which the PA1698 recombinant protein immunized-rat antisera obtained in Example 5 had been administered. Thus, the protective activity against the infection was confirmed.

### Industrial Applicability

A PA1698 protein targeted by an antibody according to the present invention is found to be extremely highly conservative among the strains regardless of serotypes and the like. Thus, the antibody according to the present invention is capable of reacting with various clinical isolates and exhibiting excellent effects as a medicament and a diagnostic agent for prevention and treatment of Pseudomonas aeruginosa infections. Moreover, the antibody according to the present invention is capable of demonstrating an effect of suppressing a lesion by Pseudomonas aeruginosa toward human airway epithelial cells, also. Therefore, the antibody is expected to be effective against chronic respiratory tract infections such as diffusepanbronchiolitis (DPB) and cystic fibrosis (CF). A vaccine composition of the present invention induces the antibody of the present invention in a body, and is thereby capable of exhibiting excellent preventive and therapeutic effects against Pseudomonas aeruginosa infections. The antibody and the vaccine composition according to the present invention can thus contribute greatly to prevention, treatment or diagnosis of Pseudomonas aeruginosa infections.

## Claims

1. An antibody or a functional fragment thereof, which binds to a PA1698 protein derived from Pseudomonas aeruginosa.

2. The antibody or the functional fragment thereof according to claim 1, wherein the PA1698 protein derived from Pseudomonas aeruginosa is a protein described in any one of (i) and (ii) below:
(i) a protein comprising an amino acid sequence represented by SEQ ID NO: 2; and
(ii) a protein comprising an amino acid sequence obtained by substitution, deletion, insertion, or addition of one or more amino acids in the amino acid sequence represented by SEQ ID NO: 2, the protein being functionally equivalent to a protein including the amino acid sequence represented by SEQ ID NO: 2.

3. The antibody or the functional fragment thereof according to claim 1, which binds to a surface of Pseudomonas aeruginosa.

4. The antibody or the functional fragment thereof according to claim 1, which has an activity to suppress a cytotoxic activity of Pseudomonas aeruginosa toward a human airway epithelial cell.

5. The antibody or the functional fragment thereof according to claim 1, which has an antibacterial activity in a patient infected with Pseudomonas aeruginosa.

6. The antibody or the functional fragment thereof according to claim 5, which has an antibacterial activity against a Pseudomonas aeruginosa infection in a patient with a reduced neutrophil level.

7. The antibody or the functional fragment thereof according to claim 1, which binds to an epitope of an antibody produced by a hybridoma deposited under any one of accession numbers FERM BP-11055 and FERM BP-11056.

8. A hybridoma deposited under any one of accession numbers FERM BP-11055 and FERM BP-11056.

9. A vaccine composition for use in prevention or treatment of a disease associated with Pseudomonas aeruginosa, the vaccine composition comprising: an antigen composition including any one of a protein antigen and a peptide antigen which are capable of inducing production of an antibody against a PA1698 protein derived from Pseudomonas aeruginosa; and optionally at least one pharmaceutically acceptable carrier, diluent, and/or adjuvant.

10. The vaccine composition according to claim 9, wherein the PA1698 protein derived from Pseudomonas aeruginosa is a protein described in any one of (i) and (ii) below:
(i) a protein comprising the amino acid sequence represented by SEQ ID NO: 2; and
(ii) a protein comprising an amino acid sequence obtained by substitution, deletion, insertion, or addition of one or more amino acids in the amino acid sequence represented by SEQ ID NO: 2, the protein being functionally equivalent to a protein including the amino acid sequence represented by SEQ ID NO: 2.

11. The vaccine composition according to claim 9, wherein the disease associated with Pseudomonas aeruginosa is a systemic infectious disease caused by a Pseudomonas aeruginosa infection.

12. A pharmaceutical composition for use in prevention or treatment of a disease associated with Pseudomonas aeruginosa, the pharmaceutical composition comprising: the antibody or the functional fragment thereof according to any one of claims 1 to 7; and optionally at least one pharmaceutically acceptable carrier and/or diluent.

13. The pharmaceutical composition according to claim 12, wherein the disease associated with Pseudomonas aeruginosa is a systemic infectious disease caused by a Pseudomonas aeruginosa infection.

14. A diagnostic agent for a Pseudomonas aeruginosa infection, comprising the antibody or the functional fragment thereof according to any one of claims 1 to 3.

15. A kit for detecting Pseudomonas aeruginosa, comprising the antibody or the functional fragment thereof according to any one of claims 1 to 3.


SEQUENCE LISTING

<110>  Meiji Seika Kaisha, Ltd.

<120>  Pseudomonas aeruginosa Type III secretion system related protein P
A1698

<130>  PM1927

<150>  JP 2007-331653
<151>  2007-12-25

<160>  11

<170>  PatentIn version 3.1

<210>  1
<211>  867
<212>  DNA
<213>  Pseudomonas aeruginosa

<400>  1
atggacatcc tccagagttc ctccgccgcg cccctcgcgc cgcgggaagc ggccaacgcc      60

cccgcgcagc aggctggcgg cagctttcag ggcgagcgcg tccactacgt ttccgtttcg     120

cagtcgctgg ccgatgccgc ggaagagctg accttcgcct tttccgagcg cgccgagaaa     180

tccctggcca agcgccgcct gagcgacgcc catgcgcgcc tgagcgaagt ccaggccatg     240

ctgcaggagt actggaagcg cattccggac ctggaaagcc agcagaagct cgaggcgctg     300

atcgcccacc tgggcagcgg ccaactgagc agcctggcgc agctcagcgc ttacctggag     360

ggcttctcca gcgagatcag ccagcgcttc ctggcgctgt cgcgggcgcg cgacgtcctg     420

gccgggcggc cggaggcgcg ggcgatgctg gcgctggtcg accaggcgtt gctgcggatg     480

gccgacgagc agggcctgga gatcgaactc ggcctgcgca tcgagccgct ggccgccgag     540

gcttccgccg ccggtgtcgg cgatatccag gcgctgcgcg atacctaccg tgacgcggta     600

cttgactacc ggggcctgtc ggcggcctgg caggacatcc aggcgcgctt cgccgcgacg     660

ccgctggagc gggtcgtggc cttcctgcag aaagccctga gcgccgacct ggacagtcag     720

tcgagccggc tcgatccggt gaagctggag cgggtcatga gcgacatgca caagctgcgc     780

gtgctcggcg gcctggcgga gcaggtgggg gcgctctggc aggtgctggt gacaggggag     840

cggggccatg gcatacgggc cttctga                                          867

<210>  2
<211>  288
<212>  PRT
<213>  Pseudomonas aeruginosa

<400>  2

Met Asp Ile Leu Gln Ser Ser Ser Ala Ala Pro Leu Ala Pro Arg Glu
1               5                   10                  15

Ala Ala Asn Ala Pro Ala Gln Gln Ala Gly Gly Ser Phe Gln Gly Glu
            20                  25                  30

Arg Val His Tyr Val Ser Val Ser Gln Ser Leu Ala Asp Ala Ala Glu
        35                  40                  45

Glu Leu Thr Phe Ala Phe Ser Glu Arg Ala Glu Lys Ser Leu Ala Lys
    50                  55                  60

Arg Arg Leu Ser Asp Ala His Ala Arg Leu Ser Glu Val Gln Ala Met
65                  70                  75                  80

Leu Gln Glu Tyr Trp Lys Arg Ile Pro Asp Leu Glu Ser Gln Gln Lys
                85                  90                  95

Leu Glu Ala Leu Ile Ala His Leu Gly Ser Gly Gln Leu Ser Ser Leu
            100                 105                 110

Ala Gln Leu Ser Ala Tyr Leu Glu Gly Phe Ser Ser Glu Ile Ser Gln
        115                 120                 125

Arg Phe Leu Ala Leu Ser Arg Ala Arg Asp Val Leu Ala Gly Arg Pro
    130                 135                 140

Glu Ala Arg Ala Met Leu Ala Leu Val Asp Gln Ala Leu Leu Arg Met
145                 150                 155                 160

Ala Asp Glu Gln Gly Leu Glu Ile Glu Leu Gly Leu Arg Ile Glu Pro
                165                 170                 175

Leu Ala Ala Glu Ala Ser Ala Ala Gly Val Gly Asp Ile Gln Ala Leu
            180                 185                 190

Arg Asp Thr Tyr Arg Asp Ala Val Leu Asp Tyr Arg Gly Leu Ser Ala
        195                 200                 205

Ala Trp Gln Asp Ile Gln Ala Arg Phe Ala Ala Thr Pro Leu Glu Arg
    210                 215                 220

Val Val Ala Phe Leu Gln Lys Ala Leu Ser Ala Asp Leu Asp Ser Gln
225                 230                 235                 240

Ser Ser Arg Leu Asp Pro Val Lys Leu Glu Arg Val Met Ser Asp Met
                245                 250                 255

His Lys Leu Arg Val Leu Gly Gly Leu Ala Glu Gln Val Gly Ala Leu
            260                 265                 270

Trp Gln Val Leu Val Thr Gly Glu Arg Gly His Gly Ile Arg Ala Phe
        275                 280                 285

<210>  3
<211>  25
<212>  DNA
<213>  artificial sequence

<220>
<223>  primer

<400>  3
ggcgcatccg gacgatgaga ggaga                                          25

<210> 4
<211> 25
<212> DNA
<213> artificial sequence

<220>
<223> primer

<400> 4
ctcgagcaag gcgatgactg cgccg 25


<210> 5
<211> 20
<212> DNA
<213> artificial sequence

<220>
<223> primer

<400> 5
ccgagaaatc cctggccaag 20


<210> 6
<211> 20
<212> DNA
<213> artificial sequence

<220>
<223> primer

<400> 6
acagcgccag gaagcgctgg 20


<210> 7
<211> 20
<212> DNA
<213> artificial sequence

<220>
<223> primer

<400> 7
gatcgaactc ggcctgcgca 20

<210> 8
<211> 29
<212> DNA
<213> artificial sequence

<220>
<223> primer

<400> 8
aaggaaaaaa gcggccgcat ggacatcct 29


<210> 9
<211> 25
<212> DNA
<213> artificial sequence

<220>
<223> primer

<400> 9
cgcggatcct cagaaggccc gtatg 25


<210> 10
<211> 31
<212> DNA
<213> artificial sequence

<220>
<223> primer

<400> 10
cagtcagtca tatggacatc ctccagagtt c 31


<210> 11
<211> 19
<212> DNA
<213> artificial sequence

<220>
<223> primer

<400> 11
gctagttatt gctcagcgg 19